# EUROPÄISCHE PATENTANMELDUNG

(11) **EP 3 214 067 A1**
(43) Veröffentlichungstag der Anmeldung: **06.09.2017**
(21) Anmeldenummer: 17158957.5
(22) Anmeldetag: 02.03.2017
(51) Int. Cl.: C07C 69/67, C07C 69/75

(54) **VERFAHREN ZUR AUFREINIGUNG VON CYCLOHEXANPOLYCARBONSÄUREESTERN**

(30) Priorität: 03.03.2016 EP 16158452
(71) Anmelder: BASF SE, 67056 Ludwigshafen am Rhein (DE)
(72) Erfinder: BITTERLICH, Stefan, 67246 Dirmstein (DE); SCHRAUT, Armin, 64625 Bensheim (DE); GAITZSCH, Friedemann, 67117 Limburgerhof (DE); HAAS, Michael, 68799 Reilingen (DE); HAMMON, Ulrich, 68163 Mannheim (DE)
(74) Vertreter: Reitstötter Kinzebach

(57) **Zusammenfassung**

Die vorliegende Erfindung betrifft ein Verfahren zur Aufreinigung von Cyclohexanpolycarbonsäureestern. Das Verfahren kann kontinuierlich oder batchweise durchgeführt werden.

## Beschreibung

### HINTERGRUND DER ERFINDUNG

Die vorliegende Erfindung betrifft ein Verfahren zur Aufreinigung von Cyclohexanpolycarbonsäureestern. Das Verfahren kann kontinuierlich oder batchweise durchgeführt werden.

### STAND DER TECHNIK

Cyclohexanpolycarbonsäureester stellen wichtige Wertstoffe dar und eignen sich u. A. hervorragend als Weichmacher in Kunststoffen, wie beispielsweise PVC.

Es besteht zunehmend Bedarf, die bislang überwiegend als Weichmacher in Kunststoffen eingesetzten Benzolpolycarbonsäureester zu ersetzen, da insbesondere die Phthalat-Weichmacher im Verdacht stehen, gesundheitsschädlich zu sein. Dies gilt vor allem für Kunststoffe, die in sensiblen Anwendungsbereichen, wie Kinderspielzeug, Lebensmittelverpackungen oder medizinischen Artikeln, zum Einsatz kommen. Da Cyclohexanpolycarbonsäureester im Gegensatz zu ihren unhydrierten aromatischen Analoga toxikologisch unbedenklich sind und daher auch in sensiblen Anwendungsbereichen eingesetzt werden können, eignen sie sich besonders gut als alternative Weichmacher in sensiblen Anwendungsbereichen. Zudem weisen Cyclohexanpolycarbonsäureester im Vergleich zu ihren unhydrierten aromatischen Analoga in der Regel eine niedrigere Dichte und Viskosität auf, was oft zu einer verbesserten Verarbeitbarkeit und/oder zu besseren mechanischen Eigenschaften der weichgemachten Kunststoffe führt.

Im Stand der Technik sind diverse Verfahren zur Herstellung von Cyclohexanpolycarbonsäureestern bekannt.

Beispielsweise beschreibt die WO 99/32427 ein Verfahren zur Herstellung von Cyclohexanpolycarbonsäureestern, bei dem Benzolpolycarbonsäureester mit einem Wasserstoff enthaltenden Gas in Gegenwart eines Hydrierkatalysators hydriert werden. Unter anderem beschreibt die WO 99/32427 konkret die Herstellung von 1,2-Cyclohexandicarbonsäurediisononylester, bei dem Phthalsäuredüsononylester (Palatinol N) mit Wasserstoff in Gegenwart eines Aluminiumoxid-geträgerten Ru-Katalysators hydriert wird und das nach der Hydrierung erhaltene Rohprodukt einer Wasserdampfdestillation bei 170 °C und einem Druck von 50 mbar unterzogen wird, um leichtsiedende Verunreinigungen, wie beispielsweise etwaige bei der Hydrierung entstandenen Nebenprodukte, zu entfernen.

Bei der Verwendung von Wasserdampf als Strippgas (Wasserdampfdestillation) zur Reinigung von Cyclohexanpolycarbonsäureestern, wie 1,2-Cyclohexandicarbonsäurediisononylester, fällt zwar nur ein geringer Anteil an Abgas an, da der die Verunreinigungen enthaltende Wasserdampf in der Regel problemlos großtechnisch kondensiert werden kann. Allerdings ist das hierbei anfallende Wasserdampfkondensat mit organischen Stoffen verunreinigt und muss einer Abwasser-Nachbehandlung, beispielsweise einer biologischen Abwasserreinigung oder einer Ozonierung, unterzogen werden. Des Weiteren ist der für die Reinigung der Cyclohexanpolycarbonsäureester geeignete Druckbereich durch den bei der Kondensation vorliegenden Wasserdampfdruck nach unten limitiert. Dies schränkt die durch die Wasserdampfdestillation erzielbare Produktreinheit ein und bedingt entsprechend niedrige Temperaturen des zur Kondensation des Wasserdampfs verwendeten Kühlmediums.

Es besteht daher Bedarf an einem verbesserten Verfahren zur Aufreinigung von Cyclohexanpolycarbonsäureester-Rohprodukten, wie sie beispielsweise nach der Hydrierung der entsprechenden aromatischen Vorläufer erhaltenen werden, bei dem die oben beschriebenen Nachteile, die sich durch die Verwendung von Wasserdampf als Strippgas (Wasserdampfdestillation) ergeben, überwunden werden können.

Der vorliegenden Erfindung liegt somit die Aufgabe zugrunde, ein effizientes und wirtschaftliches Verfahren zur Aufreinigung von Cyclohexanpolycarbonsäureester-Rohprodukten, wie sie beispielsweise nach der Hydrierung der entsprechenden Benzolpolycarbonsäureester-Vorläufer erhalten werden, bereitzustellen, bei dem die auf die Benzolpolycarbonsäureester einwirkende thermische Belastung als auch die Menge der bei dem Verfahren entstehenden Abgase und/oder Abwässer (Kondensate), die der Aufbereitung bzw. der Abfallbeseitigung zugeführt werden müssen, möglichst gering gehalten werden soll. Das Verfahren sollte sich zudem für eine kontinuierliche Verfahrensführung eignen.

Diese Aufgabe wird gelöst durch ein Verfahren zur Aufreinigung von Cyclohexanpolycarbonsäureestern, bei dem man
i) ein Cyclohexanpolycarbonsäureester-haltiges Rohprodukt bereitstellt,
ii) das in Schritt i) bereitgestellte Cyclohexanpolycarbonsäureester-haltige Rohprodukt einer thermischen Aufreinigung in wenigstens einem Stoffaustauschapparat durch Einleiten eines Gasstroms G1 im Sumpf des wenigstens einen Stoffaustauschapparats unterzieht, unter Erhalt eines an dem Cyclohexanpolycarbonsäureester angereicherten Sumpfprodukts SP und eines an wenigstens einer Verunreinigung angereicherten Gasstroms AG1,
wobei der Gasstrom G1 wenigstens ein unter den Verfahrensbedingungen nicht kondensierbares Gas oder Gasgemisch sowie maximal 30 Gew.-% Wasserdampf enthält, und wobei man den Gasstrom AG1 vollständig oder teilweise einer Kondensationsvorrichtung zuführt, zur zumindest teilweisen Kondensation der in dem Gasstrom AG1 enthaltenen wenigstens einen Verunreinigung, unter Erhalt eines an der wenigstens einen Verunreinigung abgereicherten Gasstroms AG2, den man, gegebenenfalls zusammen mit dem restlichen Teil des Gasstroms AG1, als Gasstrom G1 wieder in den Sumpf des wenigstens einen Stoffaustauschapparats einleitet.

Ein weiterer Gegenstand der Erfindung betrifft ein Verfahren, wie oben definiert, bei dem das in Schritt i) bereitgestellte Cyclohexanpolycarbonsäureester-haltige Rohprodukt durch Hydrierung wenigstens eines Benzolpolycarbonsäureesters unter Verwendung eines wasserstoffhaltigen Gases in Gegenwart eines Hydrierkatalysators erhalten wird.

Zudem wurde gefunden, dass die Reinheit und/oder Ausbeute der Cyclohexanpolycarbonsäureester gesteigert werden kann, wenn der Cyclohexanpolycarbonsäureesterhaltige Sumpf in dem wenigstens einen Stoffaustauschapparat gekühlt wird.

Ein weiterer Gegenstand der Erfindung betrifft daher ein Verfahren, wie oben definiert, bei dem während der thermischen Aufreinigung in Schritt ii) der Cyclohexanpolycarbonsäureester-haltige Sumpf in dem wenigstens einen Stoffaustauschapparat gekühlt wird.

Ein weiterer Gegenstand der Erfindung betrifft ein Verfahren, wie oben definiert, bei dem der Gasstrom G1 vor der Einleitung in den wenigstens einen Stoffaustauschapparat erwärmt wird.

Ein weiterer Gegenstand der Erfindung betrifft die kontinuierliche Durchführung des Verfahrens, wie im Folgenden definiert.

### BESCHREIBUNG DER ERFINDUNG

Das erfindungsgemäße Verfahren weist wenigstens einen der folgenden Vorteile auf:
- Das Verfahren ermöglicht die Herstellung von Cyclohexanpolycarbonsäureestern in hoher Reinheit.
- Mit dem erfindungsgemäßen Verfahren kann die Menge der bei dem Verfahren entstehenden Abgase und/oder Abwässer (Kondensate), die der Aufbereitung bzw. der Abfallbeseitigung zugeführt werden müssen, gering gehalten werden.
- Das Verfahren ist Aufgrund der vorteilhaften verfahrenstechnischen Gestaltung einfach und im großtechnischen Maßstab durchführbar.
- Das Verfahren kann in vorteilhafter Weise kontinuierlich durchgeführt werden.
- Zudem zeichnet sich das erfindungsgemäße Verfahren aufgrund der oben genannten Merkmale durch eine vorteilhafte Effizienz und Wirtschaftlichkeit aus.

### Schritt i):

Das in dem erfindungsgemäßen Verfahren als Einsatzstoff verwendete Cyclohexanpolycarbonsäureester-haltige Rohprodukt enthält üblicherweise neben wenigstens einem Cyclohexanpolycarbonsäureester auch einen gewissen Anteil an destillierbaren Verunreinigungen, die einen relativ niedrigen Siedepunkt, beispielsweise einen Siedepunkt bei 50 mbar von weniger als 200 °C, aufweisen und/oder die sich mit geeigneten Schleppmitteln aus dem Cyclohexanpolycarbonsäureester-haltigen Rohprodukt austreiben lassen.

Bei dem wenigstens einen Cyclohexanpolycarbonsäureester, der in dem Cyclohexanpolycarbonsäureester-haltigen Rohprodukt enthalten ist, handelt es sich in der Regel um Mono-, Di- oder gegebenenfalls Tri- oder Tetraester der 1,2-Cyclohexandicarbonsäure, 1,3-Cyclohexandicarbonsäure, 1,4-Cyclohexandicarbonsäure, 1,2,4-Cyclohexantricarbonsäure, 1,3,5-Cyclohexantricarbonsäure und der 1,2,4,5,-Cyclohexantetracarbonsäure, wobei die Estergruppen unabhängig voneinander ausgewählt sind unter Alkyl- oder Cycloalkylestern.

Bevorzugt handelt es sich bei dem wenigstens einen Cyclohexanpolycarbonsäureester, der in dem Cyclohexanpolycarbonsäureester-haltigen Rohprodukt enthalten ist, um Mono-, Di- oder gegebenenfalls Triester der 1,2-Cyclohexandicarbonsäure, 1,3-Cyclohexandicarbonsäure, 1,4-Cyclohexandicarbonsäure und der 1,2,4-Cyclohexantricarbonsäure, wobei die Estergruppen unabhängig voneinander ausgewählt sind unter Alkyl- oder Cycloalkylestern.

Die in den zuvor genannten Alkylestern enthaltenen Alkylgruppen weisen in der Regel unabhängig voneinander 3 bis 20, besonders bevorzugt 4 bis 18, insbesondere 4 bis 15 Kohlenstoffatome auf und können unverzweigt oder verzweigt sein.

Die in den zuvor genannten Cycloalkylestern enthaltenen Cycloalkylgruppen weisen in der Regel 4 bis 10, insbesondere 5 bis 7 Kohlenstoffatome auf.

Bei den bevorzugten 1,2-Cyclohexandicarbonsäureestern handelt es sich beispielsweise um 1,2-Cyclohexandicarbonsäurebutylcyclohexylester,
1,2-Cyclohexandicarbonsäuredi-n-pentylester,
1,2-Cyclohexandicarbonsäuremonocyclohexylester,
1,2-Cyclohexandicarbonsäuredicyclohexylester,
1,2-Cyclohexandicarbonsäuredi-n-hexylester,
1,2-Cyclohexandicarbonsäurediisohexylester,
1,2-Cyclohexandicarbonsäuredi-n-heptylester,
1,2-Cyclohexandicarbonsäurediisoheptylester,
1,2-Cyclohexandicarbonsäure-n-octyl-n-hexylester,
1,2-Cyclohexandicarbonsäuredi-n-octylester,
1,2-Cyclohexandicarbonsäurediisoctylester,
1,2-Cyclohexandicarbonsäuremono-(2-ethylhexyl)-ester,
1,2-Cyclohexandicarbonsäuredi-(2-ethylhexyl)-ester,
1,2-Cyclohexandicarbonsäuredi-n-nonylester,
1,2-Cyclohexandicarbonsäurediisononylester,
1,2-Cyclohexandicarbonsäuredi-n-decylester,
1,2-Cyclohexandicarbonsäurediisodecylester,
1,2-Cyclohexandicarbonsäuredipropylheptylester,
1,2-Cyclohexandicarbonsäuredi-n-undecylester,
1,2-Cyclohexandicarbonsäurediisoundecylester,
1,2-Cyclohexandicarbonsäurediisododecylester,
1,2-Cyclohexandicarbonsäuredi-n-tridecylester,
1,2-Cyclohexandicarbonsäurediisotridecylester,
1,2-Cyclohexandicarbonsäurediisotetradecylester,
1,2-Cyciohexandicarbonsäurediispentadecyiester.

Bei den bevorzugten 1,3-Cyclohexandicarbonsäureestern handelt es sich beispielsweise um
1,3-Cyclohexandicarbonsäurebutylcyclohexylester,
1,3-Cyclohexandicarbonsäuredi-n-pentylester,
1,3-Cyclohexandicarbonsäuremonocyclohexylester,
1,3-Cyclohexandicarbonsäuredicyclohexylester,
1,3-Cyclohexandicarbonsäuredi-n-hexylester,
1,3-Cyclohexandicarbonsäurediisohexylester,
1,3-Cyclohexandicarbonsäuredi-n-heptylester,
1,3-Cyclohexandicarbonsäurediisoheptylester,
1,3-Cyclohexandicarbonsäure-n-octyl-n-hexylester,
1,3-Cyclohexandicarbonsäuredi-n-octylester,
1,3-Cyclohexandicarbonsäurediisoctylester,
1,3-Cyclohexandicarbonsäuremono-(2-ethylhexyl)-ester,
1,3-Cyclohexandicarbonsäuredi-(2-ethylhexyl)-ester,
1,3-Cyclohexandicarbonsäuredi-n-nonylester,
1,3-Cyclohexandicarbonsäurediisononylester,
1,3-Cyclohexandicarbonsäuredi-n-decylester,
1,3-Cyclohexandicarbonsäurediisodecylester,
1,3-Cyclohexandicarbonsäuredipropylheptylester,
1,3-Cyclohexandicarbonsäuredi-n-undecylester,
1,3-Cyclohexandicarbonsäurediisoundecylester,
1,3-Cyclohexandicarbonsäurediisododecylester,
1,3-Cyclohexandicarbonsäuredi-n-tridecylester,
1,3-Cyclohexandicarbonsäurediisotridecylester,
1,3-Cyclohexandicarbonsäurediisotetradecylester,
1,3-Cyclohexandicarbonsäurediispentadecylester.

Bei den bevorzugten 1,4-Cyclohexandicarbonsäureestern handelt es sich beispielsweise um
1,4-Cyclohexandicarbonsäurebutylcyclohexylester,
1,4-Cyclohexandicarbonsäuredi-n-pentylester,
1,4-Cyclohexandicarbonsäuremonocyclohexylester,
1,4-Cyclohexandicarbonsäuredicyclohexylester,
1,4-Cyclohexandicarbonsäuredi-n-hexylester,
1,4-Cyclohexandicarbonsäurediisohexylester,
1,4-Cyclohexandicarbonsäuredi-n-heptylester,
1,4-Cyclohexandicarbonsäurediisoheptylester,
1,4-Cyclohexandicarbonsäure-n-octyl-n-hexylester,
1,4-Cyclohexandicarbonsäuredi-n-octylester,
1,4-Cyclohexandicarbonsäurediisoctylester,
1,4-Cyclohexandicarbonsäuremono-(2-ethylhexyl)-ester,
1,4-Cyclohexandicarbonsäuredi-(2-ethylhexyl)-ester,
1,4-Cyclohexandicarbonsäuredi-n-nonylester,
1,4-Cyclohexandicarbonsäurediisononylester,
1,4-Cyclohexandicarbonsäuredi-n-decylester,
1,4-Cyclohexandicarbonsäurediisodecylester,
1,4-Cyclohexandicarbonsäuredipropylheptylester,
1,4-Cyclohexandicarbonsäuredi-n-undecylester,
1,4-Cyclohexandicarbonsäurediisoundecylester,
1,4-Cyclohexandicarbonsäurediisododecylester,
1,4-Cyclohexandicarbonsäuredi-n-tridecylester,
1,4-Cyclohexandicarbonsäurediisotridecylester,
1,4-Cyclohexandicarbonsäurediisotetradecylester,
1,4-Cyclohexandicarbonsäurediispentadecylester.

Bei den bevorzugten 1,2,4-Cyclohexantricarbonsäureestern handelt es sich beispielsweise um
1,2,4-Cyclohexantricarbonsäuretri-n-pentylester,
1,2,4-Cyclohexantricarbonsäuretri-n-hexylester,
1,2,4-Cyclohexantricarbonsäuretriisohexylester,
1,2,4-Cyclohexantricarbonsäuretri-n-heptylester,
1,2,4-Cyclohexantricarbonsäuretriisoheptylester,
1,2,4-Cyclohexantricarbonsäuretri-n-octylester,
1,2,4-Cyclohexantricarbonsäuretriisoctylester,
1,2,4-Cyclohexantricarbonsäuremono-(2-ethylhexyl)-ester,
1,2,4-Cyclohexantricarbonsäuredi-(2-ethylhexyl)-ester,
1,2,4-Cyclohexantricarbonsäuretri-(2-ethylhexyl)-ester,
1,2,4-Cyclohexantricarbonsäuretri-n-nonylester,
1,2,4-Cyclohexantricarbonsäuretriisononylester,
1,2,4-Cyclohexantricarbonsäuretri-n-decylester,
1,2,4-Cyclohexantricarbonsäuretriisodecylester,
1,2,4-Cyclohexantricarbonsäuretripropylheptylester,
1,2,4-Cyclohexantricarbonsäuretri-n-undecylester,
1,2,4-Cyclohexantricarbonsäuretriisoundecylester,
1,2,4-Cyclohexantricarbonsäuretriisododecylester,
1,2,4-Cyclohexantricarbonsäuretri-n-tridecylester,
1,2,4-Cyclohexantricarbonsäuretriisotridecylester,
1,2,4-Cyclohexantricarbonsäuretriisotetradecylester,
1,2,4-Cyclohexantricarbonsäuretriispentadecylester.

Besonders bevorzugt handelt es sich bei dem wenigstens einen Cyclohexanpolycarbonsäureester, der in dem Cyclohexanpolycarbonsäureester-haltigen Rohprodukt enthalten ist, um Diester der 1,2-Cyclohexandicarbonsäure und der 1,4-Cyclohexandicarbonsäure, wobei die Estergruppen unabhängig voneinander ausgewählt sind unter unverzweigten oder verzweigten Alkylgruppen mit 5 bis 15 Kohlenstoffatomen.

Insbesondere handelt es sich bei dem wenigstens einen Cyclohexanpolycarbonsäureester, der in dem Cyclohexanpolycarbonsäureester-haltigen Rohprodukt enthalten ist, um
1,2-Cyclohexandicarbonsäurediisoheptylester,
1,2-Cyclohexandicarbonsäuredi-n-octylester,
1,2-Cyclohexandicarbonsäuredi-(2-ethylhexyl)-ester,
1,2-Cyclohexandicarbonsäurediisononylester,
1,2-Cyclohexandicarbonsäuredipropylheptylester,
1,4-Cyclohexandicarbonsäurediisoheptylester,
1,4-Cyclohexandicarbonsäuredi-n-octylester,
1,4-Cyclohexandicarbonsäuredi-(2-ethylhexyl)-ester,
1,4-Cyclohexandicarbonsäurediisononylester,
1,4-Cyclohexandicarbonsäuredipropylheptylester.

Speziell handelt es sich bei dem wenigstens einen Cyclohexanpolycarbonsäureester, der in dem Cyclohexanpolycarbonsäureester-haltigen Rohprodukt enthalten ist, um 1,2-Cyclohexandicarbonsäurediisononylester, 1,4-Cyclohexandicarbonsäuredi-(2-ethylhexyl)-ester, ganz speziell um 1,2-Cyclohexandicarbonsäurediisononylester.

Der Gehalt des wenigstens einen Cyclohexanpolycarbonsäureesters in dem in Schritt i) des erfindungsgemäßen Verfahrens bereitgestellten Cyclohexanpolycarbonsäureesterhaltigen Rohprodukt liegt in der Regel bei mindestens 85 Gew.-%, bevorzugt bei mindestens 90 Gew.-%, insbesondere bei mindestens 95 Gew.-% oder höher, beispielsweise bei 97 oder 99,5 Gew.-%.

Die in den Cyclohexanpolycarbonsäureester-haltigen Rohprodukten enthaltenen Mengen an Verunreinigungen liegt in der Regel bei maximal 15 Gew.-%, bevorzugt bei maximal 10 Gew.-%, insbesondere bei maximal 5 Gew.-% oder weniger, beispielsweise bei 3 oder 0,5 Gew.-%.

In einer bevorzugten Ausführungsform des erfindungsgemäßen Verfahrens umfasst die Bereitstellung des Cyclohexanpolycarbonsäureester-haltigen Rohprodukts in Schritt i) folgende Schritte:
i.1) Bereitstellung wenigstens eines Benzolpolycarbonsäureesters,
i.2) Hydrierung des in Schritt i.1) bereitgestellten wenigstens einen Benzolpolycarbonsäureesters unter Verwendung eines wasserstoffhaltigen Gases in Gegenwart eines Hydrierkatalysators.

Die katalytische Hydrierung von Benzolpolycarbonsäureestern zu den entsprechenden Cyclohexanpolycarbonsäureestern kann nach üblichen, dem Fachmann bekannten Methoden erfolgen.

Beispielsweise kann die katalytische Hydrierung des wenigstens einen Benzolpolycarbonsäureesters mittels Wasserstoff in Gegenwart eines geträgerten Ru-Katalysators, der gegebenenfalls noch weitere Metalle der I., VII. oder VIII. Nebengruppe des Periodensystems der Elemente enthalten kann, erfolgen, wie beispielsweise in der WO 99/32427 beschrieben.

In diesem Zusammenhang wird auf die in der WO 99/32427 enthaltenen Offenbarung in vollem Umfang Bezug genommen.

Nach der Lehre der WO 99/32427 eignen sich zur Hydrierung von Polycarbonsäurediestern Katalysatoren, die als Aktivmetall mindestens ein Metall der VIII. Nebengruppe des Periodensystems alleine oder zusammen mit mindestens einem Metall der I. oder VII. Nebengruppe des Periodensystems, aufgebracht auf einem Träger, enthalten, wobei der Träger Makroporen aufweist.

Zur Hydrierung von Polycarbonsäurediestern besonders geeignet sind Katalysatoren, die als Aktivmetall mindestens ein Metall der VIII. Nebengruppe des Periodensystems alleine oder zusammen mit mindestens einem Metall der I. oder VII. Nebengruppe des Periodensystems, aufgebracht auf einem Träger, enthalten, wobei der Träger einen mittleren Porendurchmesser von mindestens 50 nm und eine BET-Oberfläche von höchstens 30 m²/g aufweist und die Menge des Aktivmetalls 0,01 bis 30 Gew.-%, bezogen auf das Gesamtgewicht des Katalysators, beträgt.

Ferner eignen sich zur Hydrierung von Polycarbonsäurediestern besonders Katalysatoren, die als Aktivmetall mindestens ein Metall der VIII. Nebengruppe des Periodensystems alleine oder zusammen mit mindestens einem Metall der I. oder VII. Nebengruppe des Periodensystems in einer Menge von 0,01 bis 30 Gew.-%, bezogen auf das Gesamtgewicht des Katalysators, aufgebracht auf einem Träger, enthalten, wobei 10 % bis 50 % des Porenvolumens des Trägers von Makroporen mit einem Porendurchmesser im Bereich von 50 nm bis 10.000 nm und 50 % bis 90 % des Porenvolumens des Trägers von Mesoporen mit einem Porendurchmesser im Bereich von 2 nm bis 50 nm gebildet werden, wobei sich die Summe der Anteile der Porenvolumina zu 100 % addiert.

Ferner eignen sich zur Hydrierung von Polycarbonsäurediestern besonders Katalysatoren, die als Aktivmetall mindestens ein Metall der VIII. Nebengruppe des Periodensystems alleine oder zusammen mit mindestens einem Metall der I. oder VII. Nebengruppe des Periodensystems in einer Menge von 0,01 bis 30 Gew.-%, bezogen auf das Gesamtgewicht des Katalysators, aufgebracht auf einem Träger, enthalten, wobei der Träger einen mittleren Porendurchmesser von mindestens 0,1 µm, und eine BET-Oberfläche von höchstens 15 m²/g aufweist.

Als Träger können prinzipiell alle Träger eingesetzt werden, die Makroporen aufweisen, d. h. Träger, die ausschließlich Makroporen aufweisen sowie solche, die neben Makroporen auch Meso- und/oder Mikroporen enthalten.

Als makroporöses Trägermaterial verwendbar sind beispielsweise Makroporen aufweisende Aktivkohle, Siliciumcarbid, Aluminiumoxid, Siliciumdioxid, Titandioxid, Zirkoniumdioxid, Magnesiumoxid, Zinkoxid oder Gemische aus zwei oder mehr davon, wobei Aluminiumoxid und Zirkoniumdioxid vorzugsweise verwendet werden.

Die zuvor genannten Begriffe "Makroporen" und "Mesoporen" sind in Pure Appl. Chem., 45, S. 79 (1976) definiert und bezeichnen Poren, deren Durchmesser oberhalb von 50 nm (Makroporen) oder deren Durchmesser zwischen 2 nm und 50 nm liegt (Mesoporen).

Als Aktivmetall können prinzipiell alle Metalle der VIII. Nebengruppe des Periodensystems eingesetzt werden. Vorzugsweise werden als Aktivmetalle Platin, Rhodium, Palladium, Cobalt, Nickel oder Ruthenium oder ein Gemisch aus zwei oder mehr davon eingesetzt, wobei insbesondere Ruthenium als Aktivmetall verwendet wird. Unter den ebenfalls verwendbaren Metallen der I. oder VII. oder aber der I. und der VII. Nebengruppe des Periodensystems, die ebenfalls allesamt prinzipiell verwendbar sind, werden vorzugsweise Kupfer und/oder Rhenium eingesetzt.

Der Gehalt des Aktivmetalls beträgt im Allgemeinen ungefähr 0,01 bis ungefähr 30 Gew.-%, vorzugsweise ungefähr 0,01 bis ungefähr 5 Gew.-% und insbesondere ungefähr 0,1 bis ungefähr 5 Gew.-%, jeweils bezogen auf das Gesamtgewicht des verwendeten Katalysators, wobei die bei den im Folgenden beschriebenen vorzugsweise eingesetzten Katalysatoren 1 bis 3 vorzugsweise verwendeten Gehalte nochmals bei der Diskussion dieser Katalysatoren einzeln angegeben sind.

Die Hydrierung wird im Allgemeinen bei einer Temperatur von ungefähr 50 °C bis 250 °C, vorzugsweise ungefähr 70 ° bis 220 °C durchgeführt. Die dabei verwendeten Drücke liegen in der Regel bei oberhalb von 10 bar, vorzugsweise ungefähr 20 bis ungefähr 300 bar.

Die Hydrierung kann entweder kontinuierlich oder diskontinuierlich durchgeführt werden, wobei die kontinuierliche Verfahrensdurchführung bevorzugt ist.

Bei der kontinuierlichen Verfahrensführung beträgt die Menge des zur Hydrierung vorgesehenen Benzolpolycarbonsäureesters bzw. des Gemischs aus zwei oder mehr davon vorzugsweise ungefähr 0,05 bis ungefähr 3 kg pro Liter Katalysator pro Stunde, weiter bevorzugt ungefähr 0,1 bis ungefähr 1 kg pro Liter Katalysator pro Stunde.

Als Hydriergase können beliebige Gase verwendet werden, die freien Wasserstoff enthalten und keine schädlichen Mengen an Katalysatorgiften, wie beispielsweise CO, aufweisen. Beispielsweise können Reformerabgase verwendet werden. Vorzugsweise wird reiner Wasserstoff als Hydriergas verwendet.

Die Hydrierung kann in Ab- oder Anwesenheit eines Lösungs- oder Verdünnungsmittels durchgeführt werden, d. h. es ist nicht erforderlich, die Hydrierung in Lösung durchzuführen.

Vorzugsweise wird bei der Hydrierung jedoch ein Lösungs- oder Verdünnungsmittel eingesetzt. Als Lösungs- oder Verdünnungsmittel kann jedes geeignete Lösungsmittel- oder Verdünnungsmittel eingesetzt werden. Die Auswahl ist dabei nicht kritisch, solange das eingesetzte Lösungs- oder Verdünnungsmittel in der Lage ist, mit dem zu hydrierenden Benzoldicarbonsäureester eine homogene Lösung zu bilden. Beispielsweise können die Lösungs- oder Verdünnungsmittel auch Wasser enthalten. Beispiele geeigneter Lösungs- oder Verdünnungsmittel, die bei der Hydrierung eingesetzt werden können, schließen die Folgenden ein:

Geradkettige oder cyclische Ether, wie beispielsweise Tetrahydrofuran oder Dioxan, sowie aliphatische Alkohole, in denen der Alkylrest vorzugsweise 1 bis 10 Kohlenstoffatome, insbesondere 3 bis 6 Kohlenstoffatome aufweist. Beispiele bevorzugt verwendbarer Alkohole sind i-Propanol, n-Butanol, i-Butanol und n-Hexanol. Gemische dieser oder anderer Lösungs- oder Verdünnungsmittel können ebenfalls verwendet werden.

Die Menge des bei der Hydrierung eingesetzten Lösungs- oder Verdünnungsmittels ist nicht in besonderer Weise beschränkt und kann je nach Bedarf frei gewählt werden, wobei jedoch solche Mengen bevorzugt sind, die zu einer 10 bis 70 gew.-%igen Lösung der (des) zur Hydrierung vorgesehenen Benzoldicarbonsäure(esters) führen.

Die nach der katalytischen Hydrierung erhaltenen Rohprodukte enthalten in der Regel, neben dem jeweiligen wenigstens einen Cyclohexanpolycarbonsäureester, auch geringe Mengen an Nebenprodukten. Beispielsweise können im Fall der Di- oder Triester auch geringe Mengen an Mono- bzw. Mono- und/oder Diester im Rohprodukt enthalten sein. Weitere gängige Verunreinigungen sind die zur Herstellung der jeweiligen Esterverbindungen verwendeten Alkohole sowie die über Kondensationsreaktionen entstandenen Etherverbindungen davon. Des Weiteren können 1,2-Cyclohexandicarbonsäureester-Rohprodukte auch geringe Mengen Hexahydrophthalid und/oder Cyclohexandicarbonsäureanhydrid enthalten.

### Schritt ii):

Schritt ii) des erfindungsgemäßen Verfahrens beinhaltet die thermische Aufreinigung des in Schritt i) bereitgestellten Cyclohexanpolycarbonsäureester-haltigen Rohprodukts in wenigstens einem Stoffaustauschapparat durch Einleiten eines Gasstroms G1 (nachfolgend auch als "Strippgas" bezeichnet) im Sumpf des wenigstens einen Stoffaustauschapparats. Die thermische Aufreinigung in Schritt ii) des erfindungsgemäßen Verfahrens wird nachfolgend auch als "Strippung" bezeichnet.

Im Rahmen der vorliegenden Erfindung wird unter dem Begriff "Strippung" ein dem Fachmann bekanntes Verfahren verstanden, bei dem ein Rohprodukt, das verdampfbare Verunreinigungen enthält, in einem geeigneten Stoffaustauschapparat mit einem die Verunreinigung zumindest teilweise aufnehmenden gasförmigen Strippmedium, auch "Strippgas" genannt, in Kontakt gebracht wird. Der Teil des Stoffaustauschapparats, in dem der Kontakt von Rohprodukt und Strippgas stattfindet, wird auch als Kontaktzone bezeichnet.

In der Regel enthält der in der thermischen Aufreinigung in Schritt ii) eingeleitete Gasstrom G1 (Strippgas) ein unter den Verfahrensbedingungen nicht kondensierbares Gas oder Gasgemisch sowie maximal 30 Gew.-% Wasserdampf.

Bei dem in dem Gasstrom G1 enthaltenen wenigstens einen unter den Verfahrensbedingungen nicht kondensierbaren Gas handelt es sich bevorzugt um Stickstoff, Kohlendioxid, Sauerstoff, Wasserstoff, Helium, Neon, Argon, Krypton und Xenon oder Mischungen davon.

Besonders bevorzugt handelt es sich bei dem in dem Gasstrom G1 enthaltenen wenigstens einen nicht kondensierbaren Gas um Stickstoff, Kohlendioxid, Sauerstoff, Wasserstoff oder Mischungen davon, insbesondere um eine Mischung aus Stickstoff und Sauerstoff, die gegebenenfalls eine kleine Menge an Wasserstoff enthält.

Der Anteil des unter den Verfahrensbedingungen nicht kondensierbaren Gases oder Gasgemisches in dem Gasstrom G1 liegt bevorzugt bei wenigstens 50 Gew.-%, besonders bevorzugt bei wenigstens 60 Gew.-% und insbesondere bei wenigstens 70 Gew.-%, bezogen auf das Gesamtgewicht des Gasstroms G1.

In einer bevorzugten Ausführungsform des erfindungsgemäßen Verfahrens besteht der zur thermischen Aufreinigung in Schritt ii) eingeleitete Gasstrom G1 im Wesentlichen aus einem unter den Verfahrensbedingungen nicht kondensierbaren Gas oder Gasgemisch, wie oben definiert, und Wasserdampf, wobei der Anteil des Wasserdampfes in dem Gasstrom G1 maximal 30 Gew.-%, bezogen auf das Gesamtgewicht des Gasstroms G1, beträgt. In diesem Zusammenhang bedeutet der Begriff "im Wesentlichen", dass der Gasstrom G1 überwiegend, d. h. zu wenigstens 80 Gew.-%, bevorzugt zu wenigstens 90 Gew.-%, insbesondere zu wenigstens 95 Gew.-%, aus einem unter den Verfahrensbedingungen nicht kondensierbaren Gas oder Gasgemisch, wie oben definiert, und Wasserdampf besteht, wobei der Anteil des Wasserdampfes in dem Gasstrom G1 maximal 30 Gew.-%, bezogen auf das Gesamtgewicht des Gasstroms G1, beträgt.

In einer bevorzugten Ausführungsform des erfindungsgemäßen Verfahrens beträgt der Wasserdampfanteil des in Schritt ii) eingeleiteten Gasstroms G1 1 bis 30 Gew.-%, besonders bevorzugt 2 bis 20 Gew.-%, insbesondere 5 bis 15 Gew.-%, bezogen auf das Gesamtgewicht des Gasstroms G1.

Im Rahmen der vorliegenden Erfindung wird unter dem Begriff "thermische Aufreinigung" ein dem Fachmann bekannter Prozess verstanden, bei dem ein Stoffgemisch entweder durch Zuführung von thermischer Energie und/oder durch Zuführung eines Gases aufgetrennt wird. Im Falle der Zuführung von thermischer Energie erfolgt die Auftrennung des Stoffgemisches aufgrund der unterschiedlichen Siedepunkte der im Stoffgemisch enthaltenen Verbindungen. Hierbei handelt es sich beispielsweise um eine destillative Auftrennung. Im Falle der Zuführung eines Gases zum Stoffgemisch erfolgt die Auftrennung im Wesentlichen durch die unterschiedliche Flüchtigkeit der einzelnen in dem Stoffgemisch enthaltenen Verbindungen. Dabei werden leichtflüchtige Verbindungen, d. h. Verbindungen, die leichter in die Gasphase übertreten können, eher mitgeschleppt als schwerflüchtige Verbindungen, d. h. Verbindungen, die nur schwer in die Gasphase übertreten können. Hierbei handelt es sich in der Regel um ein dem Fachmann bekanntes Verfahren der Strippung, wobei das zugeführte Gas als Strippgas bezeichnet wird. Oft werden bei der thermischen Aufreinigung zur Auftrennung von Stoffgemischen beide Methoden eingesetzt, d. h. dem aufzutrennenden Gemisch wird sowohl thermische Energie als auch ein Strippgas zugeführt. Bevorzugt handelt es sich bei der "thermischen Aufreinigung" in dem vorliegenden Verfahren um eine Strippung.

Im Rahmen der vorliegenden Erfindung wird unter dem Begriff "Stoffaustauschapparat" eine Vorrichtung verstanden, in der Gase und Flüssigkeiten miteinander in Kontakt gebracht werden und dabei ein Stofftransport zwischen dem Gas bzw. der Gasphase und der Flüssigkeit bzw. der Flüssigphase ermöglicht wird (Stofftransport aus dem Gas bzw. der Gasphase in die Flüssigkeit bzw. Flüssigphase und/oder aus der Flüssigkeit bzw. Flüssigphase in das Gas oder in die Gasphase). Im dem vorliegenden Verfahren handelt es sich bei dem Stoffaustauschapparat in der Regel um eine Vorrichtung, welche den Übertritt von in der Flüssigkeit bzw. der Flüssigphase befindlichen leichtflüchtigen Bestandteilen in das Gas bzw. in die Gasphase ermöglicht.

Bei dem Stoffaustauschapparat handelt es sich beispielsweise um eine Destillationsvorrichtung oder eine Strippvorrichtung.

Als Stoffaustauschapparat eignen sich in der Regel alle dem Fachmann geläufigen Vorrichtungen zur Auftrennung von Reaktionsgemischen, die flüssige Komponenten enthalten. Geeignete Vorrichtungen umfassen Kolonnen, die mit Einbauten und/oder mit Packungen, ausgerüstet sein können, Blasensäulen, Drehbandkolonnen Verdampfer, Dünnschichtverdampfer, Fallfilmverdampfer, Zwangsumlaufverdampfer, Sambay-Verdampfer, Sprühapparate, etc. und Kombinationen davon.

Besonders bevorzugt werden zur thermischen Aufreinigung in Schritt ii) Kolonnen verwendet, die mit Einbauten, wie beispielsweise mit Böden, wie Glockenböden oder Siebböden, oder mit Platten, wie Siebplatten, und/oder mit Packungen, wie beispielsweise mit Füllkörperpackungen (z. B. Pallringen und insbesondere IMTP Füllkörper oder andere Hochleistungsfüllkörper) oder strukturierten Packungen (insbesondere Mellapak Plus™), ausgerüstet sind. Geeignete und bevorzugte Ausgestaltungen der zur thermischen Aufreinigung in Schritt ii) verwendeten wenigstens einen Destillationsvorrichtung findet sich beispielsweise in Green, Don W.; Perry, Robert H. (2008). Perry's Chemical Engineer's Handbook (8th Edition) McGraw-Hill., Section 14.

Besonders bevorzugt werden zur thermischen Aufreinigung in Schritt ii) gepackte Kolonnen verwendet, die druckverlustarme Einbauten und/oder Packungen, wie insbesondere Hochleistungsstrukturpackungen aufweisen.

Die thermische Aufreinigung in Schritt ii) des erfindungsgemäßen Verfahrens kann bei Umgebungsdruck oder bei vermindertem Druck erfolgen. Bevorzugt erfolgt die thermische Aufreinigung bei vermindertem Druck.

Besonders bevorzugt erfolgt die thermische Aufreinigung in Schritt ii) des erfindungsgemäßen Verfahrens bei einem Druck im Bereich von 10 bis 200 mbar, insbesondere im Bereich von 30 bis 100 mbar.

Die Temperatur bei der thermischen Aufreinigung in Schritt ii) des erfindungsgemäßen Verfahrens liegt in der Regel im Bereich von 50 bis 250 °C, bevorzugt im Bereich von 100 bis 200 °C. Diese Temperatur entspricht der Temperatur oberhalb der Kontaktzone des wenigstens einen Stoffaustauschapparats, insbesondere der Temperatur am Kopf der wenigstens einen Kolonne.

Die Wärmezufuhr erfolgt überwiegend über das eingespeiste Cyclohexanpolycarbonsäureester-haltige Rohprodukt. Darüber hinaus kann noch, falls gewünscht, eine geringere Wärmezufuhr über den Gasstrom G1 (Strippgas) erreicht werden.

Das Cyclohexanpolycarbonsäureester-haltige Rohprodukt wird zur thermischen Aufreinigung in Schritt ii) bevorzugt im oberen Bereich, besonders bevorzugt am Kopfbereich des wenigstens einen Stoffaustauschapparats, eingespeist.

Unter dem Begriffen "Kopfbereich des Stoffaustauschapparats" wird im Rahmen der vorliegenden Erfindung der Bereich des Stoffaustauschapparats, bevorzugt der Kolonne, verstanden, der sich oberhalb der trennungswirksamen Kolonneneinbauten befindet.

Das in Schritt i) des erfindungsgemäßen Verfahrens bereitgestellte Cyclohexanpolycarbonsäureester-haltige Rohprodukt kann direkt der thermischen Aufreinigung in Schritt ii) zugeführt werden. Das Cyclohexanpolycarbonsäureester-haltige Rohprodukt kann aber auch vor der Einspeisung in den wenigstens einen Stoffaustauschapparat zwecks Entfernung der gegebenenfalls in dem Cyclohexanpolycarbonsäureesterhaltigen Rohprodukt gelösten Gase, zuerst einer Vorrichtung zur Abtrennung von in Flüssigkeiten gelöster Gase zugeführt werden. Die Abtrennung der gegebenenfalls in dem Cyclohexanpolycarbonsäureester-haltigen Rohprodukt gelösten Gase, insbesondere von Wasserstoff, erfolgt dabei über eine Druckabsenkung (auch als "Flash" bezeichnet). Dabei wird der Druck auf einen Bereich abgesenkt, der beispielsweise unterhalb des bei der Hydrierung in Schritt I.b) verwendeten Wasserstoffdrucks aber oberhalb des bei der thermischen Aufreinigung verwendeten Drucks liegt. Bevorzugt liegt der Druck zur Abtrennung der gegebenenfalls in dem Cyclohexanpolycarbonsäureester-haltigen Rohprodukts gelösten Gase im Bereich von 0,5 bis 35 bar, insbesondere im Bereich von 1 bis 10 bar.

In einer bevorzugten Ausführungsform des erfindungsgemäßen Verfahrens weist das in den wenigstens einen Stoffaustauschapparat eingespeiste Cyclohexanpolycarbonsäureester-haltige Rohprodukt eine Temperatur im Bereich von 50 bis 250 °C, bevorzugt im Bereich von 100 bis 200 °C auf.

Bevorzugt ist der zur thermischen Aufreinigung verwendete wenigstens eine Stoffaustauschapparat nach dem Gegenstromprinzip ausgestaltet. Dabei wird das Strippgas (Gasstrom G1) am unteren Ende der Kontaktzone, d. h. im Sumpf bzw. unterhalb der trennwirksamen Kolonneneinbauten des wenigstens einen Stoffaustauschapparats, eingespeist, wobei das Strippgas dann im Gegenstrom zu dem im Kopfbereich des Stoffaustauschapparats eingeführten Cyclohexanpolycarbonsäureester-haltigen Rohprodukts durch den Stoffaustauschapparat strömt.

Bei diesem Vorgang kommt das Strippgas (Gasstrom G1) mit dem flüssigen Cyclohexanpolycarbonsäureester-haltigen Rohprodukt in Kontakt und reichert sich an den darin enthaltenen wenigstens einen Verunreinigung an, wobei auch ein Teil des wenigstens einen Cyclohexanpolycarbonsäureesters mitgeschleppt werden kann. Der so erhaltene an der wenigstens einen Verunreinigung angereicherte Gasstroms (Gasstrom AG1) wird anschließend vollständig oder teilweise einer geeigneten Kondensationsvorrichtung (Kondensatoren) zugeführt, wobei wenigstens ein Teil der in dem Gasstrom AG1 enthaltenen wenigstens einen Verunreinigung kondensiert wird, unter Erhalt eines an der wenigstens einen Verunreinigung abgereicherten Gasstroms (Gasstrom AG2).

In einer bevorzugten Ausführungsform des erfindungsgemäßen Verfahrens wird der gesamte in Schritt ii) erhaltene Gasstrom AG1 einer Kondensationsvorrichtung zur zumindest teilweisen Kondensation der in dem Gasstrom AG1 enthaltenen wenigstens einen Verunreinigung zugeführt.

Bevorzugt liegt die in den wenigstens einen Stoffaustauschapparat eingeleitete Menge an Gasstrom G1 im Bereich von 0,1 bis 100 mol, besonders bevorzugt im Bereich von 0,5 bis 50 mol, insbesondere im Bereich von 1 bis 10 mol, pro Kilogramm Cyclohexanpolycarbonsäureester-haltiges Rohprodukt.

Zur Kondensation der in dem Gasstrom AG1 enthaltenen wenigstens einen Verunreinigung können alle geeigneten Kondensatoren verwendet werden. Diese können mit beliebigen Kühlmedien gekühlt werden. Kondensatoren mit Luftkühlung und/oder Wasserkühlung sind bevorzugt, wobei die Wasserkühlung besonders bevorzugt ist. Das Kühlmedium weist dabei bevorzugt eine Temperatur im Bereich von 1 bis 40 °C, insbesondere im Bereich von 1 bis 25 °C auf. In einer ersten Ausführung befindet sich der Kondensator am Kopf, d. h. am oberen Ende des Stoffaustauschapparats. In einer zweiten bevorzugten Ausführungsform ist der Kondensator als separater der Kolonne nachgeschalteter Apparat ausgestaltet.

Das Kondensat, das bei der Kondensation der in dem Gasstrom AG1 enthaltenen wenigstens einen Verunreinigung anfällt, wird ausgeschleust und einer geeigneten, vorzugsweise einer für Flüssigkeiten geeigneten, Verbrennungsvorrichtung zugeführt.

Die Kondensationsvorrichtung (Kondensator), die zur Kondensation der in dem Gasstrom AG1 enthaltenen wenigstens einen Verunreinigung verwendete wird, ist bevorzugt so ausgestaltet, dass diese unter Betriebsbedingungen einen möglichst geringen Druckverlust, beispielsweise von weniger als 10 mbar, bevorzugt von weniger als 5 mbar, besonders bevorzugt von weniger als 2 mbar, aufweist. Hierzu eignen sich speziell sogenannte Plattenwärmeübertrager, d. h. Kolonnen mit eingebauten Platten, die parallel zur Strömungsrichtung des Strippgases angeordnet sind.

Die Temperatur des nach der Kondensation verbleibenden, an der wenigstens einen Verunreinigung abgereicherten, Gasstroms AG1 liegt in der Regel bei 0 bis 100 °C, bevorzugt bei 10 bis 80 °C, besonders bevorzugt bei 30 bis 50 °C.

Der nach der Kondensation verbleibende, an der wenigstens einen Verunreinigung abgereicherte Gasstrom AG2 wird in dem erfindungsgemäßen Verfahren, gegebenenfalls zusammen mit dem Teil des Gasstroms AG1, der nicht der Kondensationsvorrichtung zugeführten wurde, wieder als Gasstrom G1 in den Sumpf des wenigstens einen Stoffaustauschapparats eingeleitet. Dabei wird der an der wenigstens einen Verunreinigung abgereicherte Gasstrom AG2, gegebenenfalls zusammen mit dem Teil des Gasstroms AG1, der nicht der Kondensationsvorrichtung zugeführt wurde, vor der Einleitung in den Sumpf des wenigstens einen Stoffaustauschapparats zuerst einer Vorrichtung zur Gasweiterleitung, beispielsweise einem Gebläse oder einem Ventilator, oder einer Vorrichtung zur Druckerhöhung, beispielsweise einem Verdichter, insbesondere einer Vorrichtung zur Druckerhöhung, zugeführt und gegebenenfalls mit einem Vorwärmer oder Aufheizer auf die zur thermischen Aufreinigung verwendete Temperatur erwärmt. Bevorzugt wird der an der wenigstens einen Verunreinigung abgereicherte Gasstrom AG2, der, gegebenenfalls zusammen mit dem Teil des Gasstroms AG1, der nicht der Kondensationsvorrichtung zugeführt wurde, als Gasstrom G1 wieder in den Sumpf des wenigstens einen Stoffaustauschapparats eingeleitet wird, auf eine Temperatur im Bereich von 50 bis 250 °C, besonders bevorzugt im Bereich von 100 bis 200 °C erwärmt.

In der Regel beträgt der Anteil des nach der Kondensation erhaltenen, an der wenigstens einen Verunreinigung abgereicherten Gasstroms AG2, der als Gasstrom G1 wieder in den Sumpf des wenigstens einen Stoffaustauschapparats eingeleitet wird, wenigstens 90 Gew.-%. Bevorzugt liegt der Anteil des nach der Kondensation verbleibenden Gasstroms AG1, der als Gasstrom G1 wieder in den Sumpf des wenigstens einen Stoffaustauschapparats eingeleitet wird, im Bereich von 90 bis 99,8 Gew.-%, besonders bevorzugt Bereich von 95 bis 99,7 Gew.-%, insbesondere im Bereich von 97 bis 99,5 Gew.-%.

Der Anteil des nach der Kondensation erhaltenen Gasstroms AG2, der nicht wieder als Gasstrom G1 in den wenigstens einen Stoffaustauschapparat zurückgeführt wird (nachfolgend auch als Abgasstrom AG3 bezeichnet), wird gegebenenfalls zunächst in eine Vorrichtung zur Druckerhöhung, beispielsweise einem Verdichter, wie eine Dampfstrahlpumpe, einer Flüssigkeitsringpumpe oder einer Kombination davon, geleitet und anschließend über ein Abgassammelsystem einer für Gase geeigneten thermischen oder katalytischen Verbrennungsvorrichtung zugeführt.

In einer bevorzugten Ausführungsform des erfindungsgemäßen Verfahrens wird während der thermischen Aufreinigung in Schritt ii) zusätzlich zu dem Gasstrom G1 ein weiterer Gasstrom G2, der im Wesentlichen wenigstens ein unter den Verfahrensbedingungen nicht kondensierbares Gas umfasst, in den wenigstens einen Stoffaustauschapparat eingeleitet.

Die Einleitung des Gasstroms G2 in den wenigstens einen Stoffaustauschapparat erfolgt in der Regel nach der Kondensation, d. h. im unteren Bereich, bevorzugt im Bereich des Sumpfes bzw. unterhalb der trennwirksamen Kolonneneinbauten, besonders bevorzugt in den Sumpf des Stoffaustauschapparats oder in den Gasstrom G1.

Bei dem Gasstrom G2 handelt es sich bevorzugt um Stickstoff, Kohlendioxid, Wasserstoff, Helium, Neon, Argon, Krypton, Xenon oder um Mischungen davon.

Besonders bevorzugt handelt es sich bei dem Gasstrom G2 um Stickstoff, Kohlendioxid, Wasserstoff oder Mischungen davon und insbesondere um Stickstoff.

Die Einleitung des weiteren Gasstroms G2 in den wenigstens einen Stoffaustauschapparat dient unter anderem dazu, die Konzentration des Sauerstoffs, der beispielsweise durch den Einfall von Leckluft in die unterhalb des Umgebungsdrucks betriebenen, zur Aufreinigung verwendeten Vorrichtung gelangen kann, gegebenenfalls unter der Sauerstoffkonzentration für die Bildung eines explosionsfähigen Gasgemisches, beispielsweise unter 10 Vol.-% oder unter 6 Vol.-%, zu halten. Falls die Sauerstoffkonzentration in irgendeinem Bereich des Gasraums der zur Aufreinigung verwendeten Vorrichtung zu hoch sein sollte, kann diese durch den zugeführten Gasstrom G2 reduziert werden. Dabei wird die Flussrate des Gasstroms G2 bevorzugt so eingestellt, dass die Sauerstoffkonzentration in jedem Bereich des Gasraums der zur Aufreinigung verwendeten Vorrichtung 10 Vol.-%, bevorzugt 8 Vol.-%, insbesondere 6 Vol.-% nicht überschreitet.

Der Druck in dem zur Auftrennung in Schritt ii) verwendeten wenigstens einen Stoffaustauschapparats kann über die Einstellung der Flussrate des Gasstroms G2 und/oder der Flussrate des Abgasstroms AG3 geregelt werden. Bevorzugt wird der Druck in dem zur Auftrennung in Schritt ii) verwendeten wenigstens einen Stoffaustauschapparat über die Einstellung der Flussrate des Abgasstroms AG3 geregelt.

Das bei der thermischen Auftrennung erhaltene, an dem wenigstens einen Cyclohexanpolycarbonsäureester angereicherten Sumpfprodukt SP sammelt sich unterhalb der Kontaktzone an. Das Sumpfprodukt kann dann unterhalb der Kontaktzone, bzw. am Sumpf des Stoffaustauschapparats, entnommen werden.

In einer weiteren besonders bevorzugten Ausführungsform des erfindungsgemäßen Verfahrens wird während der thermischen Aufreinigung in Schritt ii) der Cyclohexanpolycarbonsäureester-haltige Sumpf in dem wenigstens einen Stoffaustauschapparat gekühlt.

Dadurch soll sichergestellt werden, dass die Verweilzeit des Sumpfprodukts SP bei Temperaturen von mehr als 100 °C nach Austritt aus der Kontaktzone im Durchschnitt weniger als 20 Minuten, bevorzugt weniger als 5 Minuten, besonders bevorzugt weniger als 2 Minuten beträgt, um die erneute Bildung von Verunreinigungen, die durch thermischen Abbau des wenigstens einen Cyclohexanpolycarbonsäureesters entstehen, zu unterdrücken.

Die Kühlung des an dem wenigstens einen Cyclohexanpolycarbonsäureester angereicherten Sumpfprodukts SP kann über einen im Sumpf des Stoffaustauschapparats eingebauten Wärmetauscher erfolgen. Zur Kühlung kann das Sumpfprodukt auch mittels einer Pumpe im Kreis über einen außerhalb des Stoffaustauschapparats befindlichen Wärmetauscher geführt werden. Bevorzugt wird das Sumpfprodukt SP zur Kühlung im Kreis über einen außerhalb des Stoffaustauschapparats befindlichen Wärmetauscher geführt, wobei das Sumpfprodukt auf unter 140 °C, bevorzugt unter 100 °C, beispielsweise unter 80 oder 60 °C, gekühlt wird.

In einer weiteren besonders bevorzugten Ausführungsform des erfindungsgemäßen Verfahrens wird der Gasstrom G1 vor der Einleitung in die thermische Aufreinigung in Schritt ii) erwärmt. Die Erwärmung des Gasstroms G1 kann beispielsweise über einen Wärmetauscher oder durch eine elektrische Heizvorrichtung erfolgen.

Die thermische Aufreinigung kann sowohl in Batch- als auch in kontinuierlicher Fahrweise durchgeführt werden. Bevorzugt wird die thermische Aufreinigung in kontinuierlicher Fahrweise durchgeführt. In einer besonders bevorzugten Ausführungsform des erfindungsgemäßen Verfahrens werden die Schritte i) bzw. der Schritt i.b) als auch der Schritt ii) kontinuierlich durchgeführt.

Die Erfindung wird anhand der im Folgenden beschriebenen Beispiele und Figuren näher erläutert. Dabei sollen die Beispiele und Figuren nicht als einschränkend für die Erfindung verstanden werden.

In den nachfolgenden Beispielen und Figuren werden folgende Abkürzungen verwendet:
Nonanol steht für Isononanol, d. h. einem Gemisch aus verschiedenen Isomeren des Nonanols;
DINCH steht für 1,2-Cyclohexandicarbonsäure-diisononylester (Hexamoll® DINCH® der Firma BASF SE);
Monoester steht für 1,2-Cyclohexandicarbonsäure-mono-isononylester;
DIN-ether steht für Isononylether, die durch Wasserabspaltung aus den verschiedenen Isomeren des Nonanols gebildet werden;
H6-Phthalide steht für Hexahydro-2-benzofuran-1(3H)-on (Hexahydrophthalid).

### FIGUREN

Figur 1:

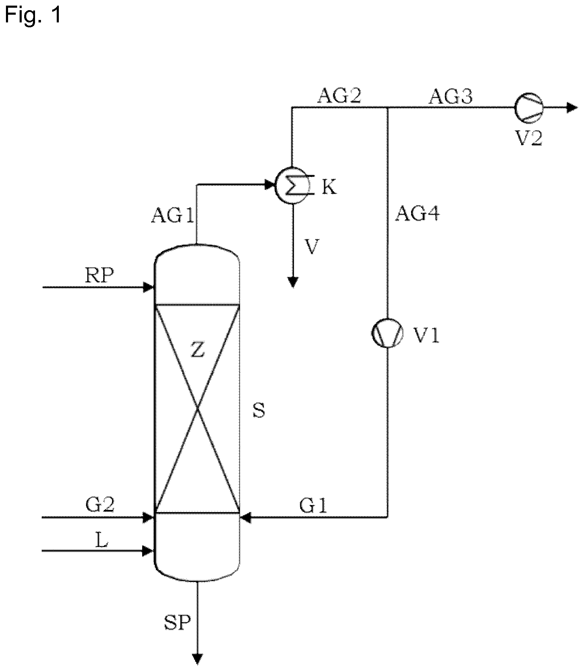

   Figur 1 zeigt eine bevorzugte Ausführungsform des erfindungsgemäßen Verfahrens. Ein Strom des Cyclohexanpolycarbonsäureester-haltigen Rohprodukts (RP) wird oberhalb der Kontaktzone (Z), welche sich innerhalb des Stoffaustauschapparats (S) befindet, geführt. Der aus dem Kopf von (S) abgezogene, an wenigstens einer Verunreinigung angereicherte Gasstrom AG1 wird in die Kondensationsvorrichtung (K) geführt, wobei ein Teil der in AG1 enthaltenen, wenigstens einen aus dem Rohprodukt entfernten Verunreinigung, auskondensiert und als flüssiger Strom (V) abgezogen wird. Aus dem nach der Kondensation verbleibenden, an der wenigstens einen Verunreinigung abgereicherten Gasstrom AG2 wird ein Teilstrom, Abgasstrom AG3, abgezogen. Der Abgasstrom AG3 wird in eine Vorrichtung zur Druckerhöhung (V2), insbesondere einen Verdichter, geführt, wodurch der Abgasstrom AG3 auf zumindest Umgebungsdruck verdichtet wird, bevor dieser einem Abgassammelsystem zugeführt wird. Der nach der Abzweigung des Abgasstroms AG3 verbleibende Reststrom von AG2 (AG4) wird in eine Vorrichtung zur Druckerhöhung (V1), beispielsweise einem Gebläse oder einen Ventilator, geführt und als Strippgas (Gasstrom G1) wieder am unteren Ende der Kontaktzone (Z), insbesondere im Sumpf, des Stoffaustauschapparats (S) zurückgeführt. Ein weiterer Gasstrom G2 wird am unteren Ende der Kontaktzone (Z) zugeführt, wobei es sich bei dem weiteren Gasstrom G2 um Stickstoff, Kohlendioxid, Wasserstoff oder um eine Mischung davon handelt. Am unteren Ende der Kontaktzone (Z), insbesondere am Sumpf der Kolonne, wird das an dem Cyclohexanpolycarbonsäureester angereicherte Sumpfprodukt SP als Flüssigstrom entnommen. Falls die thermische Auftrennung unterhalb des Umgebungsdrucks betrieben wird, ist vom Einströmen, beispielsweise an Flanschverbindungen, einer von der entsprechenden Ausführungsform abhängigen Menge an Leckluft (L) auszugehen.
Figur 2:

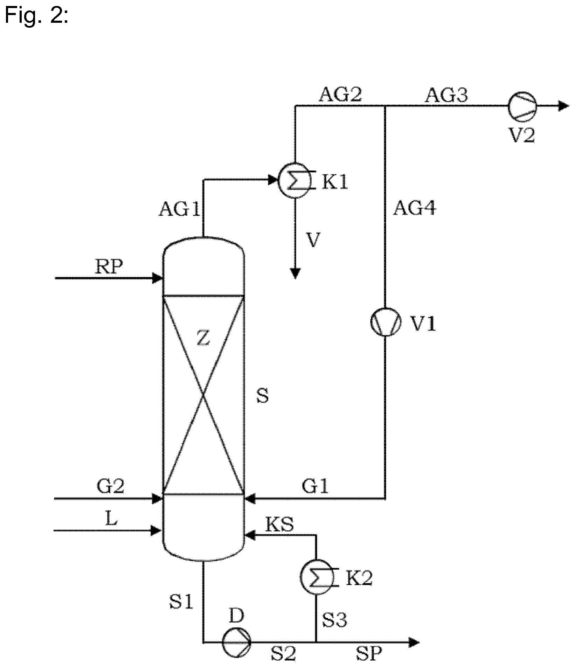

   Figur 2 zeigt eine bevorzugte Ausführungsform des erfindungsgemäßen Verfahrens, welche auf der in der Figur 1 gezeigten Ausführungsform basiert, bei dem aber zusätzlich das nach der thermischen Auftrennung erhaltene, an dem wenigstens einen Cyclohexanpolycarbonsäureester angereicherte Sumpfprodukt gekühlt wird. Diese bevorzugte Ausführungsform beinhaltet zusätzlich zu den in Figur 1 dargestellten Elementen zusätzlich die folgenden Elemente: Das nach der thermischen Auftrennung erhaltene, an dem wenigstens einen Cyclohexanpolycarbonsäureester angereicherte Sumpfprodukt wird als Sumpfstrom S1 über eine Druckerhöhungsvorrichtung (D), beispielsweise eine Pumpe, geführt. Ein Teil des aus D austretenden verdichteten Sumpfstroms S2 wird anschließend als Rückführstrom S3 (Teilstrom des verdichteten Sumpfstroms) über eine Kühlvorrichtung K2, beispielsweise einen gekühlten Wärmetauscher, geführt. Dieser wird dann als abgekühltes Sumpfprodukt KS wieder in den Sumpf des Stoffaustauschapparats (S) zurückgeführt. Der Teil des verdichteten Sumpfstroms S2, der nicht in den Sumpf des Stoffaustauschapparats (S) zurückgeführt wird, wird als gekühlter Produktstrom (SP) ausgeschleust. Dabei wird das Verhältnis des Rückführstroms (S3) zum Produktstrom (SP) so eingestellt, dass ein hohes Umlaufverhältnis erreicht wird. Dadurch wird erreicht, dass sich die im Sumpf des Stoffaustauschapparats (S) einstellende Temperatur deutlich unterhalb der Temperatur des aus der Kontaktzone (Z) austretenden, an dem Cyclohexanpolycarbonsäureester angereicherten, Produktstroms liegt. Das Verhältnis Rückführstrom (S3) : Produktstrom (SP) liegt dabei bevorzugt bei wenigstens 5 : 1, besonders bevorzugt bei wenigstens 10 : 1.
Figur 3:

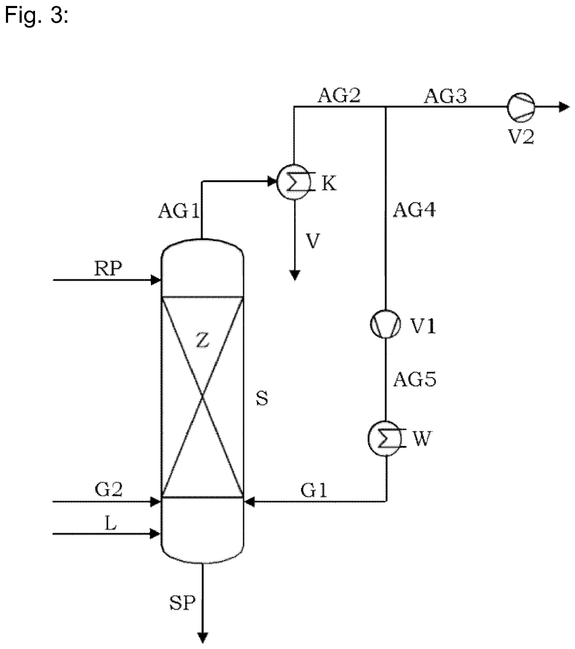

   Figur 3 zeigt eine weitere bevorzugte Ausführungsform des erfindungsgemäßen Verfahrens, welche ebenfalls auf der in der Figur 1 gezeigten Ausführungsform basiert, bei dem aber der verbleibende Gasstrom von AG2 (AG4) nach der Druckerhöhung (V1) als verdichteter Teilstrom von AG2 (AG5) zur Erwärmung über eine Wärmeübertragungsvorrichtung W, beispielsweise einem Wärmeaustauscher, geleitet wird und als Strippgas (Gasstrom G1) wieder dem Sumpf des Stoffaustauschapparats S zugeführt wird.

### BEISPIELE

Die nachfolgenden Beispiele zeigen Simulationsergebnisse des erfindungsgemäßen Verfahrens in jeweils verschiedenen Ausführungsformen. Die Simulationen wurden mittels einer BASF-eigenen Software CHEMASIM durchgeführt. Als Einsatzstoff (Rohprodukt) wurde ein Rohprodukt von 1,2-Cyclohexandicarbonsäure-diisononylester (Hexamoll® DINCH® der Firma BASF SE) angenommen, wie es nach einer typischen katalytischen Hydrierung (Kernhydrierung) des aromatischen Vorläufers (Phthalsäurediisononylester) erhalten wird. Die Art und Menge der in diesem Rohprodukt enthaltenen Verunreinigungen ist repräsentativ für diese Klasse von Esterverbindungen.

### Beispiel 1: Simulationsergebnisse für die Aufreinigung eines 1,2-Cyclohexandicarbonsäure-diisononylester-Rohproduktes (Hexamoll® DINCH® der Firma BASF SE) gemäß der in Figur 1 dargestellten Ausführungsform des erfindungsgemäßen Verfahrens:

| | **Mw** | **Einheit** | **G2** | **L** | **RP** | **G1** |
|---|---|---|---|---|---|---|
| Temperatur | | °C | 25,0 | 25,0 | 155,0 | 50,4 |
| Druck | | bar | 0,055 | 0,055 | 0,05 | 0,055 |
| mittlere Molmasse | | kg/kmol | 28,01 | 28,88 | 416,10 | 27,00 |
| Viskosität Eta | | mPa*s | 0,02 | 0,02 | 0,35 | 0,02 |
| Oberflächenspannung | | N/m | | | 0,03 | |
| Dichte | | kg/m³ | 0,06 | 0,06 | 843,87 | 0,06 |
| Mengenstrom | | kmol/h | 0,71 | 0,35 | 30,04 | 74,09 |
| Massenstrom | | kg/h | 20,0 | 10,0 | 12500,0 | 2000,0 |
| WATER | 18,02 | mol/mol | | | 0,006929 | 0,149841 |
| NONANOL | 144,26 | mol/mol | | | 0,012980 | 0,001693 |
| MONOESTER | 292,38 | mol/mol | | | 0,000996 | 0,000004 |
| DINCH | 424,67 | mol/mol | | | 0,970028 | 0,000000 |
| DIN-ETHER | 270,50 | mol/mol | | | 0,004615 | 0,000001 |
| H6-PHTHALIDE | 140,18 | mol/mol | | | 0,004452 | 0,000386 |
| N₂ | 28,01 | mol/mol | 1,000000 | 0,783421 | | 0,788074 |
| O₂ | 32,00 | mol/mol | | 0,216579 | | 0,060000 |
| WATER | 18,02 | g/g | | | 0,000300 | 0,099999 |
| NONANOL | 144,26 | g/g | | | 0,004500 | 0,009048 |
| MONOESTER | 292,38 | g/g | | | 0,000700 | 0,000047 |
| DINCH | 424,67 | g/g | | | 0,990000 | 0,000000 |
| DIN-ETHER | 270,50 | g/g | | | 0,003000 | 0,000006 |
| H6-PHTHALIDE | 140,18 | g/g | | | 0,001500 | 0,002006 |
| N₂ | 28,01 | g/g | 1,000000 | 0,760000 | | 0,817773 |
| O₂ | 32,00 | g/g | | 0,240000 | | 0,071120 |

| | **SP** | **AG1** | **V** | **AG2** | **AG4** | **AG3** |
|---|---|---|---|---|---|---|
| Temperatur | 143,2 | 154,2 | 40,0 | 40,0 | 40,0 | 40,0 |
| Druck | 0,055 | 0,05 | 0,05 | 0,05 | 0,05 | 0,05 |
| mittlere Molmasse | 423,54 | 28,33 | 188,96 | 27,00 | 27,00 | 27,00 |
| Viskosität Eta | 0,47 | 0,02 | 6,71 | 0,02 | 0,02 | 0,02 |
| Oberflächenspannung | 0,03 | | 0,03 | | | |
| Dichte | 853,99 | 0,04 | 911,68 | 0,05 | 0,05 | 0,05 |
| Mengenstrom | 29,23 | 75,96 | 0,63 | 75,34 | 74,09 | 1,25 |
| Massenstrom | 12380,0 | 2152,0 | 118,2 | 2034,0 | 2000,0 | 33,7 |
| WATER | 0,000355 | 0,148745 | 0,016744 | 0,149841 | 0,149841 | 0,149841 |
| NONANOL | 0,000718 | 0,006508 | 0,586303 | 0,001693 | 0,001693 | 0,001693 |
| MONOESTER | 0,000213 | 0,000316 | 0,037851 | 0,000004 | 0,000004 | 0,000004 |
| DINCH | 0,994254 | 0,001094 | 0,132810 | 0,000000 | 0,000000 | 0,000000 |
| DIN-ETHER | 0,003941 | 0,000309 | 0,037478 | 0,000001 | 0,000001 | 0,000001 |
| H6-PHTHALIDE | 0,000518 | 0,001938 | 0,188813 | 0,000386 | 0,000386 | 0,000386 |
| N₂ | 0,000000 | 0,781583 | 0,000000 | 0,788074 | 0,788074 | 0,788074 |
| O₂ | 0,000000 | 0,059506 | 0,000002 | 0,060000 | 0,060000 | 0,060000 |
| WATER | 0,000015 | 0,094593 | 0,001596 | 0,099999 | 0,099999 | 0,099999 |
| NONANOL | 0,000244 | 0,033142 | 0,447620 | 0,009048 | 0,009048 | 0,009048 |
| MONOESTER | 0,000147 | 0,003262 | 0,058568 | 0,000047 | 0,000047 | 0,000047 |
| DINCH | 0,996905 | 0,016398 | 0,298485 | 0,000000 | 0,000000 | 0,000000 |
| DIN-ETHER | 0,002517 | 0,002954 | 0,053652 | 0,000006 | 0,000006 | 0,000006 |
| H6-PHTHALIDE | 0,000171 | 0,009591 | 0,140079 | 0,002006 | 0,002006 | 0,002006 |
| N₂ | 0,000000 | 0,772847 | 0,000000 | 0,817773 | 0,817773 | 0,817773 |
| O₂ | 0,000000 | 0,067213 | 0,000000 | 0,071120 | 0,071120 | 0,071120 |

- RP: Rohprodukt
- SP: Sumpfprodukt
- V: Verunreinigungen (Kondensat)
- G1: Strippgas
- G2: Frischgas
- AG1: angereicherter Gasstrom
- AG2: abgereicherter Gasstrom
- AG3: Abgasstrom
- AG4: Teilstrom des abgereicherten Gasstroms
- L: Leckluft

### Beispiel 2: Simulationsergebnisse für die Aufreinigung eines 1,2-Cyclohexandicarbonsäure-diisononylester-Rohproduktes (Hexamoll® DINCH® der Firma BASF SE) gemäß der in Figur 2 dargestellten Ausführungsform des erfindungsgemäßen Verfahrens:

| | **Mw** | **Einheit** | **G2** | **L** | **RP** | **G1** |
|---|---|---|---|---|---|---|
| Temperatur | | °C | 25,0 | 25,0 | 155,0 | 50,4 |
| Druck | | bar | 0,055 | 0,055 | 0,05 | 0,055 |
| mittlere Molmasse | | kg/kmol | 28,01 | 28,88 | 416,10 | 27,01 |
| Viskosität Eta | | mPa*s | 0,02 | 0,02 | 0,35 | 0,02 |
| Oberflächenspannung | | N/m | | | 0,03 | |
| Dichte | | kg/m³ | 0,06 | 0,06 | 843,87 | 0,06 |
| Mengenstrom | | kmol/h | 0,72 | 0,35 | 30,04 | 74,05 |
| Massenstrom | | kg/h | 20,0 | 10,0 | 12500,0 | 2000,0 |
| WATER | 18,02 | mol/mol | | | 0,006929 | 0,148455 |
| NONANOL | 144,26 | mol/mol | | | 0,012980 | 0,001687 |
| MONOESTER | 292,38 | mol/mol | | | 0,000996 | 0,000004 |
| DINCH | 424,67 | mol/mol | | | 0,970028 | 0,000000 |
| DIN-ETHER | 270,50 | mol/mol | | | 0,004615 | 0,000001 |
| H6-PHTHALIDE | 140,18 | mol/mol | | | 0,004452 | 0,000386 |
| N₂ | 28,01 | mol/mol | 1,000000 | 0,783421 | | 0,789467 |
| O₂ | 32,00 | mol/mol | | 0,216579 | | 0,060000 |
| WATER | 18,02 | g/g | | | 0,000300 | 0,099026 |
| NONANOL | 144,26 | g/g | | | 0,004500 | 0,009010 |
| MONOESTER | 292,38 | g/g | | | 0,000700 | 0,000047 |
| DINCH | 424,67 | g/g | | | 0,990000 | 0,000000 |
| DIN-ETHER | 270,50 | g/g | | | 0,003000 | 0,000007 |
| H6-PHTHALIDE | 140,18 | g/g | | | 0,001500 | 0,002006 |
| N₂ | 28,01 | g/g | 1,000000 | 0,760000 | | 0,818819 |
| O₂ | 32,00 | g/g | | 0,240000 | | 0,071086 |

| | **KS** | **S1** | **AG1** | **S2** | **SP** | **S3** |
|---|---|---|---|---|---|---|
| Temperatur | 115,6 | 124,9 | 154,2 | 125,0 | 125,0 | 125,0 |
| Druck | 0,055 | 0,055 | 0,05 | 1,1 | 0,055 | 0,055 |
| mittlere Molmasse | 423,44 | 423,44 | 28,33 | 423,44 | 423,44 | 423,44 |
| Viskosität Eta | 1,04 | 0,79 | 0,02 | 0,79 | 0,79 | 0,79 |
| Oberflächenspannung | 0,03 | 0,03 | | 0,03 | 0,03 | 0,03 |
| Dichte | 876,50 | 868,99 | 0,04 | 868,93 | 868,93 | 868,93 |
| Mengenstrom | 59,04 | 88,27 | 75,92 | 88,27 | 29,23 | 59,04 |
| Massenstrom | 25000,0 | 37380,0 | 2151,0 | 37380,0 | 12380,0 | 25000,0 |
| WATER | 0,000423 | 0,000423 | 0,147381 | 0,000423 | 0,000423 | 0,000423 |
| NONANOL | 0,000918 | 0,000918 | 0,006428 | 0,000918 | 0,000918 | 0,000918 |
| MONOESTER | 0,000214 | 0,000214 | 0,000316 | 0,000214 | 0,000214 | 0,000214 |
| DINCH | 0,993939 | 0,993939 | 0,001094 | 0,993939 | 0,993939 | 0,993939 |
| DIN-ETHER | 0,003940 | 0,003940 | 0,000309 | 0,003940 | 0,003940 | 0,003940 |
| H6-PHTHALIDE | 0,000565 | 0,000565 | 0,001921 | 0,000565 | 0,000565 | 0,000565 |
| N₂ | 0,000000 | 0,000000 | 0,783039 | 0,000000 | 0,000000 | 0,000000 |
| O₂ | 0,000000 | 0,000000 | 0,059512 | 0,000000 | 0,000000 | 0,000000 |
| WATER | 0,000018 | 0,000018 | 0,093718 | 0,000018 | 0,000018 | 0,000018 |
| NONANOL | 0,000313 | 0,000313 | 0,032729 | 0,000313 | 0,000313 | 0,000313 |
| MONOESTER | 0,000148 | 0,000148 | 0,003261 | 0,000148 | 0,000148 | 0,000148 |
| DINCH | 0,996817 | 0,996817 | 0,016395 | 0,996817 | 0,996817 | 0,996817 |
| DIN-ETHER | 0,002517 | 0,002517 | 0,002953 | 0,002517 | 0,002517 | 0,002517 |
| H6-PHTHALIDE | 0,000187 | 0,000187 | 0,009507 | 0,000187 | 0,000187 | 0,000187 |
| N₂ | 0,000000 | 0,000000 | 0,774223 | 0,000000 | 0,000000 | 0,000000 |
| O₂ | 0,000000 | 0,000000 | 0,067214 | 0,000000 | 0,000000 | 0,000000 |

| | **V** | **AG2** | **AG4** | **AG3** |
|---|---|---|---|---|
| Temperatur | 40,0 | 40,0 | 40,0 | 40,0 |
| Druck | 0,05 | 0,05 | 0,05 | 0,05 |
| mittlere Molmasse | 189,53 | 27,01 | 27,01 | 27,01 |
| Viskosität Eta | 6,73 | 0,02 | 0,02 | 0,02 |
| Oberflächenspannung | 0,03 | | | |
| Dichte | 911,90 | 0,05 | 0,05 | 0,05 |
| Mengenstrom | 0,62 | 75,30 | 74,05 | 1,25 |
| Massenstrom | 117,1 | 2034,0 | 2000,0 | 33,8 |
| WATER | 0,016553 | 0,148455 | 0,148455 | 0,148455 |
| NONANOL | 0,583993 | 0,001687 | 0,001687 | 0,001687 |
| MONOESTER | 0,038285 | 0,000004 | 0,000004 | 0,000004 |
| DINCH | 0,134345 | 0,000000 | 0,000000 | 0,000000 |
| DIN-ETHER | 0,037912 | 0,000001 | 0,000001 | 0,000001 |
| H6-PHTHALIDE | 0,188911 | 0,000386 | 0,000386 | 0,000386 |
| N₂ | 0,000000 | 0,789467 | 0,789467 | 0,789467 |
| O₂ | 0,000002 | 0,060000 | 0,060000 | 0,060000 |
| WATER | 0,001573 | 0,099026 | 0,099026 | 0,099026 |
| NONANOL | 0,444508 | 0,009010 | 0,009010 | 0,009010 |
| MONOESTER | 0,059060 | 0,000047 | 0,000047 | 0,000047 |
| DINCH | 0,301022 | 0,000000 | 0,000000 | 0,000000 |
| DIN-ETHER | 0,054109 | 0,000007 | 0,000007 | 0,000007 |
| H6-PHTHALIDE | 0,139727 | 0,002006 | 0,002006 | 0,002006 |
| N₂ | 0,000000 | 0,818819 | 0,818819 | 0,818819 |
| O₂ | 0,000000 | 0,071086 | 0,071086 | 0,071086 |

- RP: Rohprodukt
- SP: Sumpfprodukt
- S1: Sumpfstrom
- S2: verdichteter Sumpfstrom
- S3: Teilstrom des verdichteten Sumpfstroms
- KS: abgekühltes Sumpfprodukt
- P: Produkt
- V: Verunreinigungen (Kondensat)
- G1: Strippgas
- G2: Frischgas
- AG1: angereicherter Gasstrom
- AG2: abgereicherter Gasstrom
- AG3: Abgasstrom
- AG4: Teilstrom des abgereicherten Gasstroms
- L: Leckluft

### Beispiel 3: Simulationsergebnisse für die Aufreinigung eines 1,2-Cyclohexandicarbonsäure-diisononylester-Rohproduktes (Hexamoll® DINCH® der Firma BASF SE) gemäß der in Figur 3 dargestellten Ausführungsform des erfindungsgemäßen Verfahrens:

| | **Mw** | **Einheit** | **G2** | **L** | **RP** | **G1** | **SP** |
|---|---|---|---|---|---|---|---|
| Temperatur | | °C | 25,0 | 25,0 | 155,0 | 100,0 | 147,8 |
| Druck | | bar | 0,055 | 0,055 | 0,05 | 0,055 | 0,055 |
| mittlere Molmasse | | kg/kmol | 28,01 | 28,88 | 416,10 | 26,99 | 423,57 |
| Viskosität Eta | | mPa*s | 0,02 | 0,02 | 0,35 | 0,02 | 0,41 |
| Oberflächenspannung | | N/m | | | 0,03 | | 0,03 |
| Dichte | | kg/m³ | 0,06 | 0,06 | 843,87 | 0,05 | 850,12 |
| Mengenstrom | | kmol/h | 0,71 | 0,35 | 30,04 | 74,09 | 29,22 |
| Massenstrom | | kg/h | 20,0 | 10,0 | 12500,0 | 2000,0 | 12380,0 |
| WATER | 18,02 | mol/mol | | | 0,006929 | 0,150183 | 0,000338 |
| NONANOL | 144,26 | mol/mol | | | 0,012980 | 0,001694 | 0,000661 |
| MONOESTER | 292,38 | mol/mol | | | 0,000996 | 0,000004 | 0,000213 |
| DINCH | 424,67 | mol/mol | | | 0,970028 | 0,000000 | 0,994349 |
| DIN-ETHER | 270,50 | mol/mol | | | 0,004615 | 0,000001 | 0,003941 |
| H6-PHTHALIDE | 140,18 | mol/mol | | | 0,004452 | 0,000387 | 0,000497 |
| N2 | 28,01 | mol/mol | 1,000000 | 0,783421 | | 0,787731 | 0,000000 |
| O2 | 32,00 | mol/mol | | 0,216579 | | 0,060000 | 0,000000 |
| WATER | 18,02 | g/g | | | 0,000300 | 0,100239 | 0,000014 |
| NONANOL | 144,26 | g/g | | | 0,004500 | 0,009055 | 0,000225 |
| MONOESTER | 292,38 | g/g | | | 0,000700 | 0,000046 | 0,000147 |
| DINCH | 424,67 | g/g | | | 0,990000 | 0,000000 | 0,996932 |
| DIN-ETHER | 270,50 | g/g | | | 0,003000 | 0,000006 | 0,002517 |
| H6-PHTHALIDE | 140,18 | g/g | | | 0,001500 | 0,002009 | 0,000165 |
| N2 | 28,01 | g/g | 1,000000 | 0,760000 | | 0,817515 | 0,000000 |
| O2 | 32,00 | g/g | | 0,240000 | | 0,071129 | 0,000000 |

| | **AG1** | **V** | **AG2** | **AG4** | **AG3** | **AG5** |
|---|---|---|---|---|---|---|
| Temperatur | 154,2 | 40,0 | 40,0 | 40,0 | 40,0 | 50,4 |
| Druck | 0,05 | 0,05 | 0,05 | 0,05 | 0,05 | 0,055 |
| mittlere Molmasse | 28,33 | 188,78 | 26,99 | 26,99 | 26,99 | 26,99 |
| Viskosität Eta | 0,02 | 6,70 | 0,02 | 0,02 | 0,02 | 0,02 |
| Oberflächenspannung | | 0,03 | | | | |
| Dichte | 0,04 | 911,69 | 0,05 | 0,05 | 0,05 | 0,06 |
| Mengenstrom | 75,97 | 0,63 | 75,34 | 74,09 | 1,25 | 74,09 |
| Massenstrom | 2152,0 | 118,6 | 2034,0 | 2000,0 | 33,7 | 2000,0 |
| WATER | 0,149081 | 0,016796 | 0,150183 | 0,150183 | 0,150183 | 0,150183 |
| NONANOL | 0,006531 | 0,586725 | 0,001694 | 0,001694 | 0,001694 | 0,001694 |
| MONOESTER | 0,000316 | 0,037741 | 0,000004 | 0,000004 | 0,000004 | 0,000004 |
| DINCH | 0,001094 | 0,132344 | 0,000000 | 0,000000 | 0,000000 | 0,000000 |
| DIN-ETHER | 0,000309 | 0,037344 | 0,000001 | 0,000001 | 0,000001 | 0,000001 |
| H6-PHTHALIDE | 0,001946 | 0,189048 | 0,000387 | 0,000387 | 0,000387 | 0,000387 |
| N2 | 0,781219 | 0,000000 | 0,787731 | 0,787731 | 0,787731 | 0,787731 |
| O2 | 0,059504 | 0,000002 | 0,060000 | 0,060000 | 0,060000 | 0,060000 |
| WATER | 0,094806 | 0,001603 | 0,100239 | 0,100239 | 0,100239 | 0,100239 |
| NONANOL | 0,033255 | 0,448348 | 0,009055 | 0,009055 | 0,009055 | 0,009055 |
| MONOESTER | 0,003264 | 0,058452 | 0,000046 | 0,000046 | 0,000046 | 0,000046 |
| DINCH | 0,016400 | 0,297709 | 0,000000 | 0,000000 | 0,000000 | 0,000000 |
| DIN-ETHER | 0,002954 | 0,053508 | 0,000006 | 0,000006 | 0,000006 | 0,000006 |
| H6-PHTHALIDE | 0,009631 | 0,140380 | 0,002009 | 0,002009 | 0,002009 | 0,002009 |
| N2 | 0,772479 | 0,000000 | 0,817515 | 0,817515 | 0,817515 | 0,817515 |
| O2 | 0,067211 | 0,000000 | 0,071129 | 0,071129 | 0,071129 | 0,071129 |

- RP: Rohprodukt
- SP: Sumpfprodukt
- V: Verunreinigungen (Kondensat)
- G1: Strippgas
- G2: Frischgas
- AG1: angereicherter Gasstrom
- AG2: abgereicherter Gasstrom
- AG3: Abgasstrom
- AG4: Teilstrom des abgereicherten Gasstroms
- AG5: verdichteter abgereicherter Gasstrom
- L: Leckluft

## Patentansprüche

1. Verfahren zur Aufreinigung von Cyclohexanpolycarbonsäureestern, bei dem man
i) ein Cyclohexanpolycarbonsäureester-haltiges Rohprodukt bereitstellt,
ii) das in Schritt i) bereitgestellte Cyclohexanpolycarbonsäureester-haltige Rohprodukt einer thermischen Aufreinigung in wenigstens einem Stoffaustauschapparat durch Einleiten eines Gasstroms G1 im Sumpf des wenigstens einen Stoffaustauschapparats unterzieht, unter Erhalt eines an dem Cyclohexanpolycarbonsäureester angereicherten Sumpfprodukts SP und eines an wenigstens einer Verunreinigung angereicherten Gasstroms AG1,
wobei der Gasstrom G1 wenigstens ein unter den Verfahrensbedingungen nicht kondensierbares Gas oder Gasgemisch sowie maximal 30 Gew.-% Wasserdampf enthält, und wobei man den Gasstrom AG1 vollständig oder teilweise einer Kondensationsvorrichtung zuführt, zur zumindest teilweisen Kondensation der in dem Gasstrom AG1 enthaltenen wenigstens einen Verunreinigung, unter Erhalt eines an der wenigstens einen Verunreinigung abgereicherten Gasstroms AG2, den man, gegebenenfalls zusammen mit dem restlichen Teil des Gasstroms AG1, als Gasstrom G1 wieder in den Sumpf des wenigstens einen Stoffaustauschapparats einleitet.

2. Verfahren nach Anspruch 1, wobei man den gesamten in Schritt ii) erhaltenen Gasstrom AG1 einer Kondensationsvorrichtung zuführt, zur zumindest teilweisen Kondensation der in dem Gasstrom AG1 enthaltenen wenigstens einen Verunreinigung.

3. Verfahren nach einem der Ansprüche 1 oder 2, wobei die Bereitstellung des Cyclohexanpolycarbonsäureester-haltigen Rohprodukts in Schritt i) folgende Schritte umfasst:
i.1) Bereitstellung wenigstens eines Benzolpolycarbonsäureesters,
i.2) Hydrierung des in Schritt i.1) bereitgestellten wenigstens einen Benzolpolycarbonsäureesters unter Verwendung eines wasserstoffhaltigen Gases in Gegenwart eines Hydrierkatalysators.

4. Verfahren nach einem der vorangehenden Ansprüche, wobei der zur thermischen Aufreinigung in Schritt ii) eingeleitete Gasstrom G1 im Wesentlichen aus einem unter den Verfahrensbedingungen nicht kondensierbaren Gas oder Gasgemisch und Wasserdampf besteht, wobei der Anteil des Wasserdampfes in dem Gasstrom G1 maximal 30 Gew.-% beträgt, bezogen auf das Gesamtgewicht des Gasstroms G1.

5. Verfahren nach einem der vorangehenden Ansprüche, wobei es sich bei dem unter den Verfahrensbedingungen nicht kondensierbaren Gas oder Gasgemisch um Stickstoff, Kohlendioxid, Sauerstoff, Wasserstoff oder um Mischungen davon handelt.

6. Verfahren nach einem der vorangehenden Ansprüche, wobei der in Schritt ii) eingeleitete Gasstrom G1 einen Wasserdampfanteil von 5 bis 15 Gew.-% aufweist, bezogen auf das Gesamtgewicht des Gasstroms G1.

7. Verfahren nach einem der vorangehenden Ansprüche, wobei der zur thermischen Aufreinigung in Schritt ii) verwendete Stoffaustauschapparat wenigstens eine gepackte Kolonne umfasst.

8. Verfahren nach einem der vorangehenden Ansprüche, wobei die thermische Aufreinigung in Schritt ii) bei einem Druck im Bereich von 10 bis 200 mbar erfolgt.

9. Verfahren nach einem der vorhergehenden Ansprüche, wobei während der thermischen Aufreinigung in Schritt ii) zusätzlich zu dem Gasstrom G1 ein weiterer Gasstrom G2, der im Wesentlichen wenigstens ein unter den Verfahrensbedingungen nicht kondensierbares Gas umfasst, in den Sumpf des wenigstens einen Stoffaustauschapparats oder in den Gasstrom G1 eingeleitet wird.

10. Verfahren nach Anspruch 9, wobei es sich bei dem Gasstrom G2 um Stickstoff, Kohlendioxid, Wasserstoff oder um Mischungen davon handelt.

11. Verfahren nach einem der Ansprüche 9 oder 10, wobei die Flussrate des Gasstroms G2 so eingestellt wird, dass die Sauerstoffkonzentration in jedem Bereich des Gasraums der zur Aufreinigung verwendeten Vorrichtung 10 Gew.-% nicht überschreitet.

12. Verfahren nach einem der vorhergehenden Ansprüche, wobei das in Schritt ii) eingesetzte Cyclohexanpolycarbonsäureester-haltige Rohprodukt eine Temperatur im Bereich von 100 bis 200 °C aufweist.

13. Verfahren nach einem der vorhergehenden Ansprüche, wobei die in den wenigstens einen Stoffaustauschapparat eingeleitete Menge an Gasstrom G1 im Bereich von 0,1 bis 100 mol pro Kilogramm Cyclohexanpolycarbonsäureesterhaltiges Rohprodukt liegt.

14. Verfahren nach einem der vorhergehenden Ansprüche, wobei während der thermischen Aufreinigung in Schritt ii) der Cyclohexanpolycarbonsäureester-haltige Sumpf des wenigstens einen Stoffaustauschapparats gekühlt wird.

15. Verfahren nach einem der vorhergehenden Ansprüche, wobei der Gasstrom G1 vor der Einleitung in den wenigstens einen Stoffaustauschapparat erwärmt wird.

16. Verfahren nach einem der vorhergehenden Ansprüche, wobei Schritt ii) in kontinuierlicher Fahrweise durchgeführt wird.
